(19) Europäisches Patentamt
European Patent Office
Office européen des brevets

(11) EP 0 625 571 B1

(12) EUROPÄISCHE PATENTSCHRIFT

(45) Veröffentlichungstag und Bekanntmachung des Hinweises auf die Patenterteilung:
**04.10.2000 Patentblatt 2000/40**

(21) Anmeldenummer: **94107323.1**

(22) Anmeldetag: **11.05.1994**

(51) Int. Cl.7: **C12P 13/04**, C12N 15/55, C12N 15/61, C12N 9/80, C12N 9/86, C12N 9/90, C12N 1/20
// (C12N1/20, C12R1:06)

# (54) Neue Mikroorganismen, deren Verwendung und Verfahren zur Herstellung von L-Alpha-Aminosäuren

Microorganisms, their use and method of production of L-alpha-amino acids

Microorganismes, leur utilisation et méthode de production d'acides aminés L-alpha

(84) Benannte Vertragsstaaten:
**AT BE CH DE DK ES FR GB GR IE IT LI LU MC NL PT SE**

(30) Priorität: **19.05.1993 DE 4316928**

(43) Veröffentlichungstag der Anmeldung:
**23.11.1994 Patentblatt 1994/47**

(73) Patentinhaber:
**Degussa-Hüls Aktiengesellschaft**
**60287 Frankfurt am Main (DE)**

(72) Erfinder:
- **Wagner, Fritz, Prof. Dr.**
  **D-38124 Braunschweig (DE)**
- **Völkel, Dirk**
  **D-38226 Salzgitter (DE)**
- **Bommarius, Andreas, Dr.**
  **D-60323 Frankfurt am Main (DE)**
- **Drauz, Karlheinz, Prof. Dr.**
  **D-63579 Freigericht (DE)**

(56) Entgegenhaltungen:
- **ANNALS OF THE NEW YORK ACADEMY OF SCIENCES, Bd.672, 1992, NEW YORK Seiten 478 - 483 PIETSCH M. ET AL. 'A new racemase for ...'**
- **ANNALS OF THE NEW YORK ACADEMY OF SCIENCES, Bd.542, 1988, NEW YORK Seiten 323 - 329 SYLDATK C. ET AL. 'Production of...'**
- **DATABASE WPI Section Ch, Week 8706, Derwent Publications Ltd., London, GB; Class B05, AN 87-040871 & JP-A-62 000 271 (DAIICHI KAGAKU YAKU) 6. Januar 1987**
- **BIOLOGICAL ABSTRACTS, vol. 90 Philadelphia, PA, US; abstract no. 061459, GROSS C ET AL 'PRODUCTION OF L TRYPTOPHAN FROM D L-5 INDOLYLMETHYLHYDANTOIN BY RESTING CELLS OF A MUTANT OF ARTHROBACTER-SPP DSM 3747' & J BIOTECHNOL, 14 (3-4). 1990. 363-376.**
- **BIOLOGICAL ABSTRACTS, vol. 86 Philadelphia, PA, US; abstract no. 123679, MOELLER A ET AL 'STEREO AND SUBSTRATE-SPECIFICITY OF A D HYDANTOINASE AND A D-N CARBAMYL-AMINO ACID AMIDOHYDROLASE OF ARTHROBACTER-CRYSTALLOPOIETES AM 2' & ENZYME MICROB TECHNOL, 10 (10). 1988. 618-625.**

EP 0 625 571 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die Erfindung betrifft neue Mikroorganismen, die bei hohen spezifischen Aktivitäten die Fähigkeit besitzen, D-, oder L-, oder D,L-5-monosubstituierte Hydantoine oder D-, oder L-, oder D,L-N-Carbamoyl-$\alpha$-Aminosäuren in die korrespondierenden, enantiomerenreinen L-$\alpha$-Aminosäuren umzusetzen.

Bislang sind zur fermentativen bzw. enzymatischen Umwandlung von N-5-monosubstituierten Hydantoinen in die enantiomerenreinen L-$\alpha$-Aminosäure zahlreiche Methoden in der Literatur beschrieben oder zum Patent angemeldet worden (s. Syldatk, C., Müller, R., Pietzsch, M., Wagner, F., MICROBIAL AND ENZYMATIC PRODUCTION OF L-AMINO AICDS FROM D,L-5-MONOSUBSTITUTED HYDANTOINS in: Biocatalytic production of amino acids and derivates (Rozell, J. D. and Wagner, F., eds.) Hanser Verlag, München, 1992, S. 129 - 176).

Yokozeki, K., Sano, K., Eguchi, C., Iwagami, H. and Mitsugi, K. (1986): OPTIMAL CONDITIONS FOR THE ENZYMATIC PRODUCTION OF L-AROMATIC AMINO ACIDS FROM THE CORRESPONDING 5-SUBSTITUTED HYDANTOINS, Agric. Biol. Chem. 51, 729 - 736 und Yokozeki, K., Hirose, Y. and Kubota, K. (1986): MECHANISM OF ASYMMETRIC PRODUCTION OF L-AROMATIC AMINO ACIDS FROM THE CORRESPONDING HYDANTOINS BY FLAVOBACTERIUM SP., Agric. Biol. Chem., 51, 737 - 746 beschreibt einen Reaktionsmechanismus für die Hydrolyse von 5-Arylalkylhydantoinen am Beispiel von 5-Benzylhydantoin mit einer aufgereinigten Hydantoinase aus Flavobacter sp. AJ-3912. Dabei wurden die relativen Geschwindigkeiten der Hin- und Rückreaktion ermittelt und gezeigt, daß die Hinreaktion von D-5-Benzylhydantoin zum N-Carbamoyl-D-phenylalanin deutlich langsamer erfolgte, als die Hydrolyse von L-5-Benzylhydantoin zum N-Carbamoyl-L-phenylalanin. Über den Mechanismus der enantioselektiven Hydrolyse von 5-Alkylhydantoinen wurde keine Aussage getroffen, da D,L-5-Methylthioethylhydantoin nur in Spuren zu N-Carbamoyl-L-Methionin umgesetzt werden konnte.

Die Aufgabe der vorliegenden Erfindung ist neue Mikroorganismen zur Verfügung zu stellen, die einfach zu kultivieren sind und Enzyme herstellen, die in der Lage sind, in verhältnismäßig großen Mengen bzw. Geschwindigkeiten L-$\alpha$-Aminosäuren aus D-, L- und/oder D,L-5-monosubstituierten Hydantoin und/oder einer D-, L- und/oder D,L-N-Carbamoyl-$\alpha$-aminosäure herzustellen. Aufgabe ist auch ein entsprechendes Verfahren zur Herstellung von L-$\alpha$-Aminosäuren sowie die Verwendung der Mikroorganismen.

Diese Aufgabe wird mit den Mikroorganismen DSM 7329 und DSM 7330 gelöst. Diese Mikroorganismen wurden am 17.11.1992 bei der DSM-Deutsche Sammlung von Mikroorganismen und Zellkulturen GmbH, Mascheroder Weg 1 B, D-3300 Braunschweig, hinterlegt, entsprechende Bescheinigungen liegen der Beschreibung bei.

Die neu isolierten und bei der Deutschen Sammlung von Mikroorganismen hinterlegten Mikroorganismen stellen neue Arten der Spezies Arthrobacter dar.

Das Verfahren zur Herstellung von L-$\alpha$-Aminosäuren durch enzymatische Umsetzung eines D-, L- und/oder D,L-5-monosubstituierten Hydantoins und/oder einer D-, L- und/oder D,L-N-Carbamoyl-$\alpha$-aminosäure wird durchgeführt, indem die Umsetzung mittels des oder der Mikroorganismen DSM 7329 und/oder DSM 7330 erfolgt.

Wenn das Verfahren mit den Mikroorganismen selbst durchgeführt wird, dann erfolgt dies vorzugsweise mit ruhenden oder abgetöteten Mikroorganismen, damit diese das Substrat nicht selbst verwerten.

Wenn das Verfahren mittels den von diesen Mikroorganismen produzierten Enzymen erfolgt, dann kann dies mit einer Maische der Mikroorganismen (z. B. erhalten mittels einer French Press oder Glasperlenmühle), einem Rohextrakt oder mit mehr oder weniger aufgereinigten Enzymen der Mikroorganismen erfolgen.

Die vorliegende Erfindung betrifft auch die Verwendung des Mikroorganismus DSM 7329 und/oder des Mikroorganismus DSM 7330 zur Anzucht von Mutanten oder Varianten dieser Mikroorganismen.

Die Anzucht von Mutanten oder Varianten dieser Mikroorganismen kann z. B. durch Selektion spontan vorkommender Mutationen erfolgen. Andere Möglichkeiten sind z. B. die Mutationen durch Einwirkungen von chemischen Stoffen und/oder radioaktiver Strahlung und/oder UV-Licht.

Die Gewinnung eines oder mehrerer Gene aus den Mikroorganismen dient z. B. der Sequenzanalyse der Gene für die Enzyme oder zur Insertion in einen Mikroorganismus, wobei durch Auswahl des Mikroorganismus, in den das Gen insertiert wird, ein Mikroorganismus erhalten werden kann, der größere Mengen des oder der für das oben beschriebene Verfahren entscheidenden Enzyme produziert. U. U. kann das Gen auch in eine tierische oder pflanzliche Zelle eingebaut werden. Der Einbau des Gens erfolgt mit den in der Mikrobiologie üblichen Methoden, z. B. über einen Vektor.

Die beschriebenen neuen Mikroorganismen stellen eine Reihe von Enzymen in solchen Mengen her, daß die Zellmasse für die oben beschriebenen Verfahren eine sehr hohe Aktivität hat. Bislang sind keine Stämme mit einer höheren Aktivität für diese Verfahren bekannt.

Das oben beschriebene Verfahren zur Herstellung von L-$\alpha$-Aminosäuren verläuft mit den Mikroorganismen der vorliegenden Erfindung bzw. deren Enzymen praktisch stereospezifisch, wobei die verschiedensten 5-substituierten Hydantoine umgesetzt werden können. Gemäß dem später wiedergegebenen Reaktionsschema werden die entsprechenden Aminosäuren erhalten. Gemäß der vorliegenden Erfindung kann der Rest im Hydantoin ein Alkylrest sein,

verzweigt sein, cyclisch sein, mit einem oder mehreren Heteroatomen substituiert sein, aromatisch sein, heteroaromatisch sein, etc. Schwierigkeiten bereiteten lediglich 5-Methylhydantoin, 5,5-disubstituierte Hydantoine, mehrfach substituierte Phenylhydantoine, wie 5-3'-Methyl-4'-aminomethylphenylhydantoin, sperrige Substituenten unmittelbar am Hydantoin, wie 5-t-Butylhydantoin sowie Reste mit geladenen Substituenten nahe am Hydantoin, wie z. B. 5-β-Carboxylethylhydantoin, 5-γ-Aminopropylhydantoin, 5-δ-Aminobutylhydantoin. Umsetzen ließen sich hingegen eine Vielzahl von Verbindungen, wie z. B. 5-substituierte Hydantoine mit dem Substituenten Isopropyl, N-Propyl, N-Butyl, Methylthioethyl, Isobutyl, Indolylmethyl, Cyclohexylmethyl, Benzyl, Naphthylmethyl, Phenyl, wobei die unterschiedlichsten Substituenten möglich sind, z. B. am Benzyl, p-Fluor, p-Chlor, p-Amino, p-Methoxy, p-Carboxyl. Dem 5-Substituenten im Hydantoin bzw. der N-Carbamoylaminosäure können somit keine grundsätzlichen Einschränkungen zugesprochen werden, da auf Grund der Vielzahl von möglichen Substituenten und der Vielzahl der möglichen Funktionalisierungen der Substituenten bzw. der Aminosäure-Reste grundsätzlich alle typischen 5-substituierten Hydantoine oder entsprechende N-Carbamoylaminosäuren als Substrat in Betracht kommen.

Im Europa Patent 0 159 866 B1 wird ein Bakterium der Gattung Arthrobacter sp. DK-200 beschrieben, welches L-α-Aminosäuren aus dem korrespondierenden 5-Arylalkylhydantoinen und/oder aus der entsprechenden N-Carbamoylaminosäure bilden kann. Hier wird lediglich die Synthese von L-Phenylalanin, L-Tryptophan und L-Tyrosin beschrieben. Über spezifische Enzymaktivitäten oder den enzymatischen Mechanismus werden keine Angaben gemacht.
Arthrobacter sp. DK-200 unterscheidet sich eindeutig von den neuen Mikroorganismen Arthrobacter sp. DSM 7329 und DSM 7330:
Arthrobacter sp. DK-200 kann Stärke nicht hydrolytisch spalten, dies ist ein entscheidendes physiologisches Merkmal zur Identifizierung der Arten innerhalb der Gattung Arthrobacter. Ferner zeigt der Stamm keine Ureaseaktivität und in Bezug auf Denitrifizierung wird der Stamm als positiv eingestuft. Ferner ist Wachstum des Stammes Arthrobacter DK-200 nicht mehr bei einer Temperatur über 33,2°C zu beobachten. Weiterhin bestehen Unterschiede physiologischer Merkmale wie in der Verwertung von Milchzucker als Kohlenstoffquelle sowie in der Säurebildung aus Zuckern (L-Arabinose negativ und Glycerin positiv) wie aus der Tabelle 1 zu ersehen ist.

Tabelle 1

| Physiologische und Bakteriologische | | | |
|---|---|---|---|
| Eigenschaften | DSM 7329 | DSM 7330 | DK 200 |
| Denitrifizierung | - | - | + |
| Hydrolytische Spaltung von Stärke | + | + | - |
| Urease | + | + | - |
| Wachstum bei | 33°C | 35°C | 17.8°C |
| nicht bei | >34°C | >36°C | >33.3°C |
| Wachstum bei [% NaCl] | 2 % | 5 % | 2 % |
| nicht bei | >3 % | >6 % | >5 % |
| Bildung von Säuren aus Zucker: | | | |
| L-Arabinose | + | + | - |
| Glycerin | - | - | + |
| Verwertung von Kohlenstoffquellen | | | |
| Milchzucker | + | + | - |

In der Patentschrift DE 37 12 539 C2 werden 3 Mikroorganismen beschrieben, DW 3 (DSM 3745), CW 4 (DSM 3746) und CW 5 (DSM 3747) die in die Gruppe der saprophytischen, aeroben Coryneforme Bakterien einzuordnen sind. Diese Mikroorganismen sind befähigt, aus den korrespondierenden 5-Arylalkylhydantoinen und N-Carbamoyl-α-aminosäuren die entsprechende aromatische L-α-Aminosäure freizusetzen. Es wird ferner die Umsetzung von D-,L-5-Methylthioethylhydantoin über D-N-Carbamoylmethionin zu L-Methionin beschrieben. Für diese Umsetzungen werden keine spezifischen Produktivitäten für die eingesetzten Organismen angegeben. Die Umsetzungen von D-N-Carbamoylaminosäuren zu L-Aminosäuren wird durch die Anwesenheit einer Racemase auf die Ebene der N-Carbamoylzwischenverbindungen begründet (DE 37 12 539, S. 2, Z. 42 - 44). In der neuesten Veröffentlichung über den

Stamm DSM 3747 wird eine Beteiligung einer D-N-Carbamoylaminosäure bei der Gewinnung von L-Aminosäuren aus den korrespondierenden D,L-5-monosubstituierten Hydantoinen nicht mit einbezogen (vgl. Syldatk, C., Müller, R., Pietzsch, M., Wagner, F., MICROBIAL AND ENZYMATIC PRODUCTION OF L-AMINO AICDS FROM D,L-5-MONOSUBSTITUTED HYDANTOINS in: Biocatalytic production of amino acids and derivates (Rozell, J. D. and Wagner, F., eds.) Hanser Verlag, München, 1992, S. 129 - 176). Die Unterscheidung der in der Patentschrift DE 37 11 539 beschriebenen Mikroorganismen DSM 3745 bis DSM 3747 gegenüber den beiden Mikroorganismen DSM 7329 und DSM 7330 ist auf physiologischer Ebene eindeutig, wie aus der Gegenüberstellung aus Tabelle 2 hervorgeht.

Tabelle 2

| Physiologische und Bakteriologische | | | | | |
|---|---|---|---|---|---|
| Eigenschaften | DSM 7329 | DSM 7330 | DSM 3745 | DSM 3746 | DSM 3747 |
| gram-Färbung | + | + | +/- | - | - |
| Oxidase | - | - | + | - | - |
| Wachstum nicht über | 34 | 36 | 37 | 35 | 35 |
| Bildung von Säuren aus Zucker | | | | | |
| L-Arabinose | + | + | + | - | + |
| D-Sorbit | + | + | - | - | - |
| Verwertung von Kohlenstoffquellen | | | | | |
| Glycerin | - | - | + | + | + |
| Stärke | - | - | + | + | + |
| Citronensäure | - | - | + | + | + |

Es sind eine Reihe von Verfahren zur Herstellung von aliphatischen L-$\alpha$-Aminosäuren, ausgehend von 5-Alkylhydantoinen oder den entsprechenden N-Carbamoylaminosäuren, bekannt.

In der Patentschrift DE 37 02 384 A1 (US-Patent 5 071 752) wird die Bildung von L-Aminosäuren aus den korrespondierenden 5-substituierten Hydantoinen am Beispiel von L-Leucin, L-Valin und L-Cystein mit dem Mikroorganismus Nocardia spec. DSM 3306 beschrieben. Jedoch wurde weder die spezifische Aktivität angegeben noch ein Mechanismus für die Hydantoinhydrolyse vorgeschlagen.

Nach dem japanischen Patent Sho 55-29678 läßt man ein Corynebacter sepedonicum auf L- oder D,L-N-Carbamoylmethionin einwirken, wobei das im Substratgemisch enthaltene L-N-Carbamoylmethionin in L-Methionin umgesetzt wird. Nach diesem Verfahren kann jedoch nur das L-enantiomere Substrat in L-Methionin überführt werden, während das D-N-Carbamoylmethionin nicht umgesetzt wird. Ebenso wird in der japanischen Patentschrift JP-5529678 ein Prozeß beschrieben, in dem aus D,L-N-Carbamoylmethionin mit einem mikrobiellen Enzym L-Methionin gebildet wird. Nach der Umsetzung ist eine Trennung von L-Methionin von dem nicht umgesetzten D-N-Carbamoylmethionin erforderlich.

Guivarch, M., Gillonnier, C. and Brunie, J.-C. (1980): OBTENTION D AMINO ACIDES OPTIQUEMENT ACTIFS A L'AIDE D'HYDANTOINASES, Bull. Soc. Chim. Fr. No. 1-2, 91 - 95, beschreibt die Bildung von L-Methionin aus D,L-5-Methylthioethylhydantoin mit Arthrobacter ureafaciens, dabei wird eine Racemisierung auf der Zwischenstufe von D- und L-N-Carbamoylmethionin angenommen. Dieser Reaktionsweg findet in den neuen Mikroorganismen DSM 7329 und DSM 7330 nicht statt.

Im japanischen Patent JP62000771 werden zwei neuartige Arthrobacter spec. DP-B-1001 und DP-B-1002 beschrieben, die aus 5-substituierten Hydantoinen und aus N-Carbamoylaminosäuren, unabhängig ob sie in der D-, der L-, der D,L-Form oder als Gemisch vorliegen, die entsprechenden L-Aminosäuren bilden. Über einen Reaktionsmechanismus wird in dieser Patentschrift keine Aussage gemacht. Diese neuartigen Mikroorganismen unterscheiden sich aber wesentlich in ihren physiologischen Merkmalen gegenüber den Organismen DSM 7329 und DSM 7330, wie aus Tabelle 3 zu erkennen ist. Außerdem bestehen deutliche Unterschiede in den spezifischen Aktivitäten zur Umsetzung von D-N-Carbamoylmethionin zu L-Methionin, wie in Tabelle 4 aufgezeigt ist.

Tabelle 3

| Physiologische und Bakteriologische Eigenschaften | | | | |
|---|---|---|---|---|
| | DSM 7329 | DSM 7330 | 1001 | 1002 |
| Hydrolytische Spaltung von Stärke | + | + | - | - |
| Wachstum bei [°C] | 33 | 35 | 30 | 30 |
| nicht über [°C] | 34 | 36 | 37 | 37 |
| Wachstum bei [% NaCl] | 2 % | 5 % | 2 % | 2 % |
| nicht über | 3 % | 6 % | 5 % | 5 % |
| Nährstoffbedarf | - | - | Biot. | Biot. |
| Verwertung v. Malonat | + | + | + | - |
| Bildung von Säure aus Zucker: | | | | |
| D-Sorbit | + | + | - | - |
| Inosit | + | + | - | - |
| Glycerin | - | - | + | + |
| Verwertung von Kohlenstoffquellen: | | | | |
| D-Sorbit | + | + | - | - |
| Glycerin | - | - | + | + |
| Adonit | - | +/- | - | - |
| Ethanol | + | + | - | - |
| β-Alanin | + | - | + | - |
| L-Ornithin | + | + | - | - |
| L-Threonin | - | + | + | + |
| L-Valin | + | - | - | - |
| Essigsäure | - | - | + | + |
| D,L-Milchsäure | - | - | + | + |
| Propionsäure | - | - | + | + |
| meso-Weinsäure | - | + | - | - |
| m-Hydroxybenzoesäure | - | - | + | + |
| p-Hydroxybenzoesäure | - | - | + | + |
| Malonsäure | + | + | + | - |
| Bernsteinsäure | - | - | + | + |
| Citronensäure | - | - | + | + |
| Gluconsäure | - | - | + | + |
| Fumarsäure | + | - | + | + |
| Harnsäure | - | + | - | - |

Die Berechnung der Spezifischen Aktivitäten ganzer Zellen erfolgte nach folgender Gleichung:

$$A_{spez.} = \frac{\text{mmol bzw. g umgesetztes Substrat}}{\text{g BFM}\cdot\text{h}}$$

Tabelle 4:

Nach der JP (B 2) Hei 4-39316 verfügt der Japanische Stamm DP-B-1001 über folgende Spezifische Aktivitäten für D-N-Carbamoylmethionin:

$$A_{spez.} = \frac{2.0\ (\text{mmol})}{10\ (\text{g BFM})\cdot 48\ (\text{h})} = 0.00417\ (\text{mmol/g BFM X h})$$

Unter Identischen Bedingungen verfügt der Stamm DSM 7330 über folgende Spezifische Aktivitäten für D-N-Carbamoylmethionin:

$$A_{spez.} = \frac{2.6\ (mmol)}{10\ (g\ BFM) \cdot 21\ (h)} = 0.0124\ (mmol/g\ BFM\ X\ h)$$

d. h. eine Steigerung der Spezifischen Aktivitäten für D-CM unter den in der o. g. JP-B, Beispiel 1 angegebenen Bedingungen um den Faktor 3.3 bezogen auf (g/g BFM X h)

Unter Verwendung eines anderen Puffers (0.1 M Na-Carbonat-Puffer, pH: 8.5) und doppelter Substratzugabe wurde eine Spez. Aktivität von:

$$A_{spez.} = \frac{5.2\ (mmol)}{10\ (g\ BFM) \cdot 21\ (h)} = 0.0248\ (mmol/g\ BFM\ X\ h)$$

ermittelt, d. h. eine weitere Steigerung auf den Faktor 6.5.

Unter veränderten Bedingungen (0.2 M Tris/HCl-Puffer, pH: 8.0, 30°C, ohne Begasung und ohne sonstige Zusätze) wurde folgende Spezifische Aktivität ermittelt:

$$A_{spez.} = \frac{4.24\ (mmol)}{10\ (g\ BFM) \cdot 9.33\ (h)} = 0.045\ (mmol/g\ BFM\ X\ h)$$

d. h. eine weitere Steigerung auf den Faktor 12!

BFM: Bakterienfeuchtmasse;

D-CM: D-N-Carbamoylmethionin

Mit der vorliegenden Erfindung erfolgte die Isolierung zweier neuer Arthrobacter spec. DSM 7329 und DSM 7330, die gegenüber dem Stand der Technik eine deutlich erhöhte Produktivität aufweisen und vorwiegend aliphatische

L-Aminosäuren, wie z. B. L-Methionin aus D-, und/oder L-, und/oder D,L- eines 5-monosubstituierten Hydantoins und/oder der korrespondierenden N-Carbamoyl-aminosäure und/oder einem Gemisch aus den beiden erwähnten Substanzklassen nach dem in Schema 1 wiedergegebenen neu aufgeklärten Reaktionsmechanismus herstellen. "Alkyl" steht hier nur beispielhaft für das o. g. Substratspektrum. Die Pfeilstärke steht für die gefundenen Geschwindigkeiten.

Schema 1

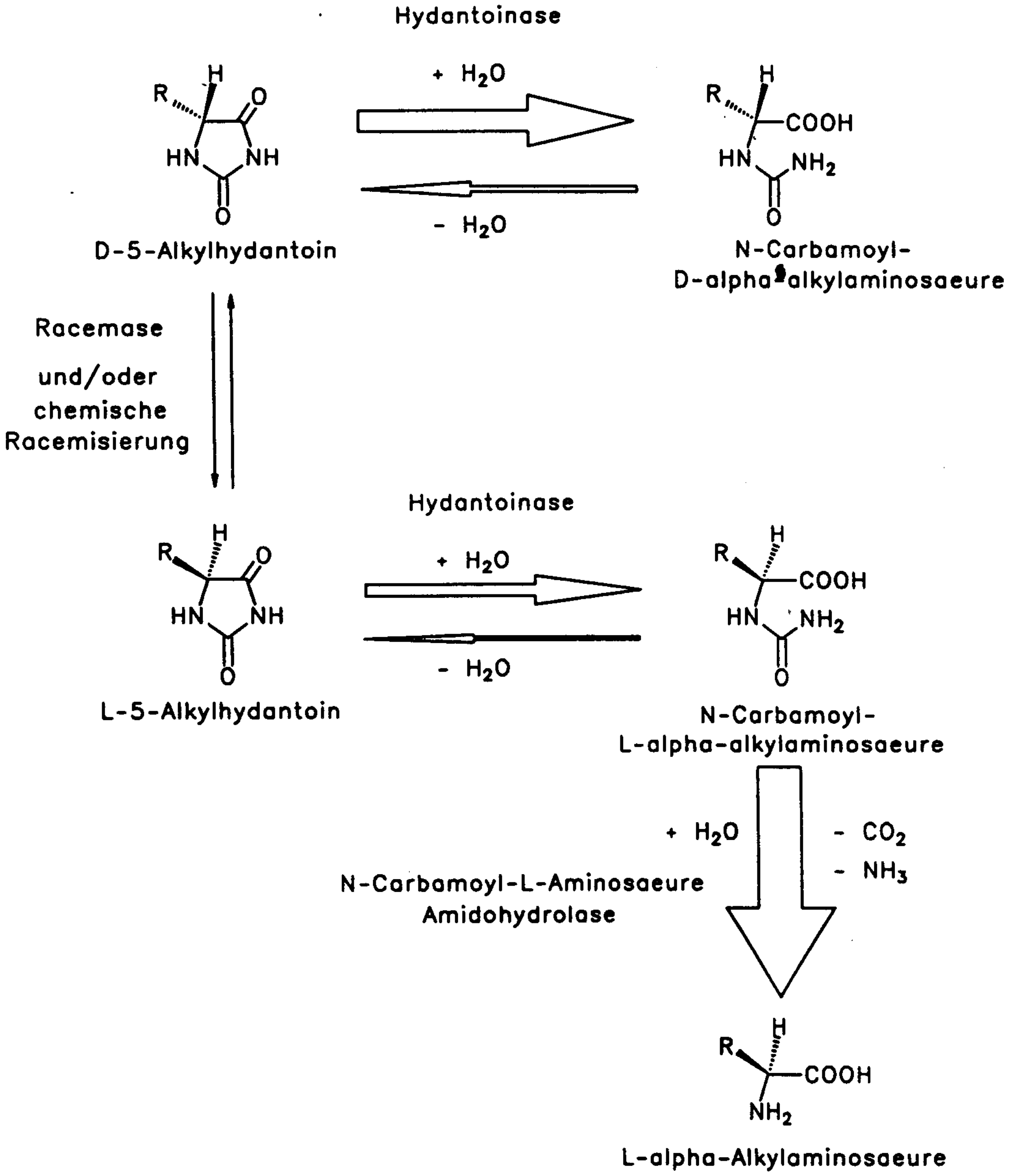


Die Erfindung wird im folgenden anhand von Beispielen näher ausgeführt.

Beispiel 1: Isolierung der Mikroorganismen

Jeweils 50 ml eines synthetischen Mediums mit D,L-5-Methylthioethylhydantoin als alleinige Stickstoffquelle der Zusammensetzung

| | |
|---|---|
| $KH_2PO_4$ | 1.0 g/l |
| $K_2HPO_4$ | 1.816 g/l |
| $MgSO_4 * 7 H_2O$ | 0.123 g/l |
| $CaCl_2 * 2 H_2O$ | 0.017 g/l |
| $FeSO_4 * 7 H_2O$ | 0.006 g/l |
| Glucose | 5.0 g/l |
| D,L-Methylthioethylhydantoin | 1.0 g/l |
| Spurenelementlsg. | 10 ml/l |
| pH: 7.0, | |

wobei die Spurenelementlösung folgende Zusammensetzung hat:

| | |
|---|---|
| Ethylendiamintetraacetat($Na^+$) | 500 mg/l |
| $FeSO_4 * 7 H_2O$ | 200 mg/l |
| $ZnSO_4 * 7 H_2O$ | 10 mg/l |
| $MnCl_2 * 4 H_2O$ | 3 mg/l |
| $H_3BO_3$ | 30 mg/l |
| $CoCl_2 * 4 H_2O$ | 1 mg/l |
| $CuCl_2 * 2 H_2O$ | 1 mg/l |
| $NiCl_2 * 6 H_2O$ | 1 mg/l |
| $Na_2MoO_4 * 2 H_2O$ | 3 mg/l, |

werden mit 1 g Bodenprobe verschiedenster Standorte in 500 ml Erlenmeyerkolben mit Schikanen bei 30°C, pH: 7,0 und 100 Upm auf eine Rotationsschüttelmaschine (Braun, Melsungen) 4 Tage inkubiert. Anschließend werden jeweils 1 ml dieser Suspension in frischer steriler der o. g. Nährlösung überimpft und erneut 4 Tage bei 30°C, pH: 7,0 und 100 Upm auf der Rotationsschüttelmaschine inkubiert. Nach 10 Kultivierungspassagen wird jeweils die Suspensionskultur über eine Verdünnungsreihe auf Agarplatten auf einem Komplexmedium ausgestrichen bis nach Inkubation bei 30°C nach 3 Tagen Einzelkolonien aufgewachsen sind. Die Einzelkolonien werden in bekannter Weise in Reinkultur gebracht.

Zusammensetzung des Komplexmediums für die einzelnen Mikroorganismen-Stämme:

| | |
|---|---|
| Glucose | 10.0 g/l |
| Techn. Hefeextrakt | 0.5 g/l |
| Zitronensäure | 0.5 g/l |
| $MgSO_4 * 7 H_2O$ | 0.1 g/l |

(fortgesetzt)

| | |
|---|---|
| $CaCl_2 * 2 H_2O$ | 0.05 g/l |
| $KH_2PO_4$ | 1.0 g/l |
| $K_2HPO_4$ | 1.816 g/l |
| Spurenelementlsg. | 10.0 ml/l |
| Agar | 20.0 g/l |
| pH: 7.0 | |

Zur Prüfung auf die enzymatische Aktivität zur Gewinnung von L-α-Aminosäuren aus D,L-5-monosubstituierten Hydantoinen werden die so erhaltenen Reinkulturen wie folgt in Submerskulturen gezüchtet:
In einem 500 ml Erlenmeyerkolben mit Schikanen werden 100 ml steriles Nährmedium I mit einer Abschwemmung einer neu erhaltenen Reinkultur beimpft und bei 30°C, pH: 7,0 und 100 Upm auf der Rotationsschüttelmaschine 24 h lang inkubiert. Von der so erhaltenen Suspenionskultur werden 500 ml steriles Nährmedium II in einem 2000 ml Erlenmeyerkolben mit Schikanen mit 10 ml der Vorkultur beimpft. Die erneute Kultivierung erfolgt über 36 h bei 30°C, pH: 7,2 und bei 100 Upm auf der Rotationsschüttelmaschine. Danach werden die Zellen abzentrifugiert und zweimal mit 0.9 %-iger NaCl-Lösung gewaschen. Die so erhaltene Zellfeuchtmasse kann entweder sofort auf die enzymatische Aktivität geprüft aber auch bei -18°C bis zu mehreren Wochen zwischengelagert werden.

Nährmedium I setzt sich wie folgt zusammen:

| | |
|---|---|
| Glucose | 10.0 g/l |
| $(NH_4)_2SO_4$ | 6.50 g/l |
| Zitronensäure | 0.32 g/l |
| $MgSO_4 * 7 H_2O$ | 0.20 g/l |
| $CaCl_2 * 2 H_2O$ | 0.02 g/l |
| $MnCl_2 * 4 H_2O$ | 0.02 g/l |
| $FeSO_4 * 7 H_2O$ | 0.02 g/l |
| $KH_2PO_4$ | 4.54 g/l |
| $K_2HPO_4$ | 7.61 g/l |

Das Nährmedium II hat folgende Zusammensetzung:

| | |
|---|---|
| Glucose | 15.0 g/l |
| Techn. Hefeextrakt | 1.0 g/l |
| $(NH_4)_2SO_4$ | 6.50 g/l |
| Zitronensäure | 0.62 g/l |
| $MgSO_4 * 7 H_2O$ | 0.40 g/l |
| $CaCl_2 * 2 H_2O$ | 0.04 g/l |
| $MnCl_2 * 4 H_2O$ | 0.04 g/l |
| $FeSO_4 * 7 H_2O$ | 0.04 g/l |
| $KH_2PO_4$ | 3.40 g/l |
| $Na_2HPO_4 * 2 H_2O$ | 4.42 g/l |

(fortgesetzt)

| | |
|---|---|
| D,L-5-Indolylmethyl-N-3-methylhydantoin | 0.25 g/l |

Die enzymatische Aktivität der so erhaltenen Zellfeuchtmasse (BFM) wird unter folgenden Reaktionsbedingungen vergleichend getestet: In 50 ml 0.2 molaren Tris/HCl-Puffer pH: 8.0 werden 50 mg D,L-5-Methylthioethylhydantoin und 2,5 g BFM zugesetzt und 24 h bei 30°C inkubiert. Danach wird die Zellsuspension zentrifugiert und der klare Überstand mittels HPLC-Analytik untersucht. Als besonders aktiv haben sich zwei Stämme herausgestellt, die bei der Deutschen Sammlung für Mikroorganismen (DSM) unter den Nummern DSM 7329 und DSM 7330 hinterlegt sind. Sie haben folgende physiologische und bakteriologische Eigenschaften, die in Tabelle 5 zusammengestellt sind.

Tabelle 5:

Physiologische und Bakteriologische Eigenschaften

| | DSM 7329 | DSM 7330 |
|---|---|---|
| Eigenschaften | | |
| Zellgröße (L·Ø) | (0.3 - 0.6) * (0.1 - 0.2)µm | |
| Zellform | kurze Stäbchen | |
| Pleomorphie | + | + |
| Beweglichkeit | - | - |
| Geißeln | - | - |
| Gram-Färbung | + | + |
| Sporen | - | - |
| Säurebeständigkeit | - | - |
| Reduktion von Nitraten | - | - |
| Denitrifizierung | - | - |
| MR-Test (Maillard-Reaktion) | - | - |
| VP-Test (Voges-Proskauer-Test) | - | - |
| Hydrolytische Spaltung von Stärke | + | + |
| Verwertung von anorganischen Stickstoffquellen | + | + |

Physiologische und Bakteriologische Eigenschaften

| | DSM 7329 | DSM 7330 |
|---|---|---|
| Bildung von Farbstoffen | - | - |
| Urease | + | + |
| Oxidase | - | - |
| Katalase | + | + |

Physiologische und Bakteriologische Eigenschaften

| | DSM 7329 | DSM 7330 |
|---|---|---|
| Wachstum bei [°C] | 33 | 35 |
| nicht über [°C] | 34 | 36 |
| Wachstum bei [% NaCl] | 2 % | 5 % |
| nicht über | 3 % | 6 % |
| Verhalten gegenüber | | |
| Sauerstoff (aerob +) | + | + |
| Nährstoffbedarf | - | - |
| Gluconsäureoxidation | - | - |
| Verwertung v. Malonat | + | + |
| | | |
| Bildung von Säure aus Zucker: | | |
| L-Arabinose | + | + |
| D-Xylose | + | + |
| D-Glucose | + | + |
| D-Mannose | + | + |
| D-Fructose | + | + |
| D-Galactose | + | + |
| Maltose | + | + |
| Rohrzucker | + | + |
| Milchzucker | + | + |
| Trehalose | + | + |
| D-Sorbit | + | + |
| D-Mannit | + | + |
| Inosit | + | + |
| Glycerin | - | - |
| Stärke | - | - |
| Raffinose | + | + |
| Adonit | - | - |
| Rhamnose | + | + |

Physiologische und Bakteriologische Eigenschaften

| | DSM 7329 | DSM 7330 |
|---|---|---|
| Verwertung von Kohlenstoffquellen: | | |
| L-Arabinose | + | + |
| D-Xylose | + | + |
| D-Glucose | + | + |
| D-Mannose | + | + |
| D-Fruçtose | + | + |
| D-Galactose | + | + |
| Malzzucker | + | + |
| Rohrzucker | + | + |
| Milchzucker | + | + |
| Threhalose | + | + |
| D-Sorbit | + | + |
| D-Mannit | + | + |
| Inosit | + | + |
| Glycerin | - | - |
| Stärke | - | - |
| Raffinose | + | + |

Physiologische und Bakteriologische Eigenschaften

| | DSM 7329 | DSM 7330 |
|---|---|---|
| Adonit | - | +/- |
| Rhamnose | + | + |
| Ethanol | + | + |
| β-Alanin | + | - |
| D,L-Arginin | + | + |
| L-Histidin | + | + |

Physiologische und Bakteriologische Eigenschaften

| | DSM 7329 | DSM 7330 |
|---|---|---|
| L-Ornithin | + | + |
| L-Threonin | - | + |
| L-Valin | + | - |
| Ameisensäure | - | - |
| Essigsäure | - | - |
| Butyrat | - | - |
| D,L-Milchsäure | - | - |
| Propionsäure | - | - |
| meso-Weinsäure | - | + |
| D-(-)-Weinsäure | - | - |
| m-Hydroxybenzoesäure | - | - |
| p-Hydroxybenzoesäure | - | - |
| Glycolsäure | - | - |
| Malonsäure | + | + |
| Bernsteinsäure | - | - |
| Citronensäure | - | - |
| α-Ketoglutarat | + | + |
| Pyruvat | + | + |
| Glyoxylat | - | - |
| Gluconsäure | - | - |
| Fumarsäure | + | - |
| Pimelinsäure | - | - |
| Harnsäure | - | + |
| Hippursäure | + | + |
| Adipinsäure | - | - |
| Glutarsäure | - | - |
| Panthothensäure | - | - |
| Lävulinsäure | - | - |
| Citraconsäure | - | - |

| Physiologische und Bakteriologische Eigenschaften | DSM 7329 | DSM 7330 |
|---|---|---|
| Azelainsäure | - | - |
| Itaconsäure | - | - |
| Betain | + | + |
| L-Methionin | - | - |
| L-Tryptophan | - | - |
| L-Phenylalanin | - | - |

Durch chemische und/oder physikalische Mutation oder durch Selektion in kontinuierlicher Kulturführung können aus diesen Mikroorganismen Mutanten oder Varianten angereichert werden mit erhöhten und/oder stabileren Enzymaktivitäten.

Beispiele für die Umsetzungen von D,L-5-monosubstituierten Hydantoinen, die mit den erfindungsgemäßen Mikroorganismen über die N-Carbamoyl-α-Aminosäuren zu L-α-Aminosäuren entsprechend dem gezeigten Reaktionsmechanismus hydrolysiert werden können, sind in Tabelle 6 aufgeführt, mit beiden Stämmen werden im Bereich der Meßgenauigkeit die gleichen Ergebnisse gefunden.

Tabelle 6:

| R = | Einsatz [mMol] | D,L-Hydantoin [mMol] | N-Carbamoyl-derivat [mMol) | L-α-Amino-säure [mMol] |
|---|---|---|---|---|
| $CH_3-S-CH_2-CH_2-$ | 11.5 | - | - | 11.4 |
| 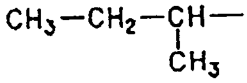 | 12.8 | 0.64 | 3.84 | 8.3 |
| 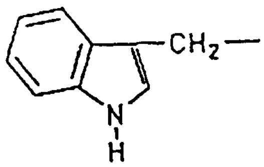 | 8.7 | - | - | 8.6 |
| 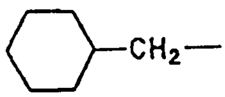 | 10.2 | 1.02 | 3.06 | 6.12 |
| 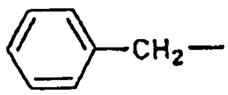 | 10.5 | - | - | 10.4 |
| 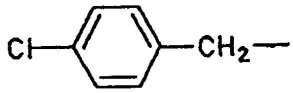 | 8.9 | - | - | 8.8 |

-: nicht nachweisbar

Reaktionsbedingungen: Die angegebene Substratkonzentration wird in 50 ml 0.1 M Natrium-Carbonat-Puffer pH: 8.5 mit 1.5 g BFH bei 30°C über 24 h inkubiert.

Beispiel 2: Anzucht der Biomasse

Ein 500 ml Erlenmeyerkolben mit 100 ml sterilem Nährmedium I wird mit einer Platinöse des Stammes DSM 7330 von einer Schrägagarkultur beimpft und auf der Rundschüttelmaschine bei 100 Upm und 30°C kultiviert. Nach einer Inkubationszeit von 24 h dient diese Suspensionskultur als Inoculum für die 2. Vorkultur. Zehn 2000 ml Erlenmeyerkolben mit 500 ml sterilem Nährmedium I werden je mit 10 ml der 1. Vorkultur angeimpft und erneut 24 h bei 100 Upm und bei 30°C inkubiert. Nach der Inkubationszeit dient diese Vorkultur II als Inokulum für einen 50 l Bioreaktor. Dieser ist ausgerüstet mit einem Intensorsystem und wird mit 45 l Nährmedium entsprechend der Zusammensetzung des

Nährmediums II versetzt, bei pH: 6,5 und einer Temperatur von 121°C und ca. 100 kPa (1 bar) Überdruck 30 min sterilisiert. Nach Abkühlen auf 30°C wird mit 10 %iger NaOH auf pH 7,0 eingestellt und anschließend mit der Suspensionskultur aus Vorkultur II angeimpft. Die Züchtung erfolgt bei 30°C und einer Rührerdrehzahl von 400 Upm und einer Belüftungsrate von 0.8 V/V/m. Nach 18 h wird die Zellfeuchtmasse abzentrifugiert und anschließend mit 0.9 %iger Kochsalzlösung zweimal gewaschen. Die so erhaltene Zellfeuchtmasse kann sofort, oder nach Zwischenlagerung bei -18°C zu einem späteren Zeitpunkt für die enzymatische Umsetzung verwendet werden. Es werden 1.725 kg Zellfeuchtmasse erhalten.

Analog wird der Stamm DSM 7329 gezüchtet, dabei werden 1,69 kg Zellfeuchtmasse, entsprechend 0.34 kg Zelltrockenmasse, erhalten.

Beispiel 3:

83 g Zellfeuchtmasse von DSM 7330 und 20 g (115 mM) D,L-Methylthioethylhydantoin werden in 1000 ml 0.1 M Soerensen-Puffer pH: 8.0 suspendiert und bei 35°C, bei $N_2$-Begasung in einem 2 l Rührreaktor unter Rühren 28 h inkubiert. Anschließend werden die Zellen abzentrifugiert und der Überstand mittels Hochdruckflüssigkeitschromatographie (HPLC) analysiert. Im Überstand wurden 16.4 g (110 mM) L-Methionin nachgewiesen.
Ausbeute = 95.6 % d. Th.

Beispiel 4:

100 g Zellfeuchtmasse von DSM 7330 und 10 g (52 mmol) D-Carbamoylmethionin werden in 1000 ml 0.2 M Tris/HCl-Puffer, pH 8.0 bei 30°C suspendiert und ohne Begasung in einem 2 l Rührreaktor unter Rühren 9 h inkubiert. Nach Zentrifugation werden im Überstand 6.6 g (44 mmol) L-Methionin nachgewiesen.
Ausbeute = 84.6 % d. Th.

Beispiel 5:

100 g Zellfeuchtmasse von DSM 7329 und 10 g (52 mmol) L-Carbamoylmethionin werden in 1000 ml 0.2 M Tris/HCl-Puffer, pH 8.0, 30°C suspendiert und ohne Begasung in einem 2 l Rührreaktor unter Rühren 3,5 h inkubiert. Nach Zentrifugation werden im Überstand 7.68 g (51.5 mmol) L-Methionin nachgewiesen.
Ausbeute = 99 % d. Th.

Beispiel 6:

100 g Zellfeuchtmasse von DSM 7330 und 10 g (52 mmol) D,L-Carbamoylmethionin werden in 1000 ml 0.2 M Tris/HCl-Puffer, pH 8.0, 30°C suspendiert und ohne Begasung in einem 2 l Rührreaktor unter Rühren 9 h inkubiert. Nach Zentrifugation werden im Überstand 7.16 g (48 mmol) L-Methionin nachgewiesen.
Ausbeute = 92 % d. Th.

Beispiel 7:

100 g Zellfeuchtmasse von DSM 7330 und 10 g Technisches Hydantoin, bestehend aus 10.4 mmol D,L-Carbamoylmethionin und 41.6 mmol D,L-Methylthioethylhydantoin, werden in 1000 ml 0.2 M Tris/HCl-Puffer, pH 8.0, 30°C suspendiert und ohne Begasung in einem 2 l Rührreaktor unter Rühren 15 h inkubiert. Nach Zentrifugation werden im Überstand 7.01 g (47 mmol) L-Methionin nachgewiesen.
Ausbeute = 90.3 % d. Th.

Beispiel 8: Herstellung eines Rohextraktes und gereinigter Enzymlösung

Die Mikroorganismen DSM 7329 bzw. 7330 werden in einer Glasperlenmühle ca. 15 min aufgeschlossen und anschließend die Biomasse durch Zentrifugieren abgetrennt. Der Überstand ist der Enzym-Rohextrakt und enthält ca. 21 g/l Protein.

Zur Isolierung der in dem Rohextrakt enthaltenen Hydantoinase wird dieser nach üblichen Methoden aufgearbeitet. Im vorliegenden Fall erfolgt dies durch aufsteigende fraktionierte Fällung, beginnend mit 1,7 M Ammoniumsulfat, anschließender Protaminsulfat(3 g/l)-Trennung und anschließender Fällung des Proteins durch 2,5 M Ammoniumsulfat. Das so erhaltene vorgereinigte Produkt wird säulenchromatographisch weiter gereinigt, wobei nacheinander Butylsepharose, Phenylsepharose und zweimal Anionenaustausch-Chromatographie (pH 7,8, pH 6,6) erfolgt. Der Stamm DSM 7329 hat nach der 1. Anionaustausch-Chromatographie einen Proteingehalt von 0,12 g/l, der Stamm

DSM 7330 nach der 2. Anionenaustausch-Chromatographie einen Proteingehalt von 0,1 g/l (nach Bredford). Die Proteinreinheit wird durch Gelelektrophorese bestimmt.

Der Enzym-Rohextrakt (21 g/l) und die gereinigte Enzymlösung (0,1 g/l Hydantoinase) werden in den folgenden Beispielen eingesetzt.

Beispiel 9:

0.00575 mmol D-Methylthioethylhydantoin wurden mit 100 l Enzym in 900 l 0.1 M Tris/HCl-Puffer pH 8.5 bei 42°C 1 h inkubiert. Nach Beendigung des Aktivitätstests wurden im Überstand 0.00351 mmol D-Carbamoylmethionin gemessen, was einem Umsatz von 61 Mol% entspricht.

Beispiel 10:

0.00575 mmol L-Methylthioethylhydantoin wurden mit 100 l Enzym in 900 l 0.1 M Tris/HCl-Puffer pH 8.5 bei 42°C 1 h inkubiert. Nach Beendigung des Aktivitätstests wurden im Überstand 0.000863 mmol L-Carbamoylmethionin gemessen, was einem Umsatz von 15 Mol% entspricht.

Beispiel 11:

0.00521 mmol D-Carbamoylmethionin wurden mit 100 l Enzym in 900 l 0.1 M Tris/HCl-Puffer pH 8.5 bei 42°C 1 h inkubiert. Nach Beendigung des Aktivitätstests wurden im Überstand 0.000162 mmol D-Methylthioethylhydantoin gemessen, was einem Umsatz von 3.1 Mol% entspricht.

Beispiel 12:

0.00521 mmol L-Carbamoylmethionin wurden mit 100 l Enzym in 900 l 0.1 M Tris/HCl-Puffer pH 8.5 bei 42°C 1 h inkubiert. Nach Beendigung des Aktivitätstests wurden im Überstand 0.0000521 mmol L-Methylthioethylhydantoin gemessen, was einem Umsatz von 1.0 Mol% entspricht.

Beispiel 13:

0.00521 mmol L-Carbamoylmethionin wurden mit 100 l Rohextrakt in 900 l 0.1 M Tris/HCl-Puffer pH 8.5 bei 42°C 0.5 h inkubiert. Nach Beendigung des Aktivitätstests wurden im Überstand 0.00521 mmol L-Methionin gemessen.

## Patentansprüche

1. Mikroorganismus DSM 7329

2. Mikroorganismus DSM 7330.

3. Verfahren zur Herstellung von L-α-Aminosäuren durch enzymatische Umsetzung eines D-, L- und/oder D,L-5-monosubstituierten Hydantoins und/oder einer D-, L-und/oder D,L-N-Carbamoyl-α-aminosäure,
**dadurch gekennzeichnet,**
daß die Umsetzung mittels des oder der Mikroorganismen DSM 7329 und/oder DSM 7330 erfolgt.

4. Verfahren nach Anspruch 3,
**dadurch gekennzeichnet,**
daß die Umsetzung mit ruhenden Mikroorganismen erfolgt.

5. Verwendung des oder der Mikroorganismen DSM 7329 und/oder DSM 7330 zur Anzucht von Mutanten oder Varianten dieser Mikroorganismen.

## Claims

1. Microorganism DSM 7329.

2. Microorganism DSM 7330.

**3.** Process for producing L-α-amino acids by enzymatic conversion of a D-, L- and/or D,L-5-monosubstituted hydantoin and/or a D-, L- and/or D,L-N-carbamoyl-α-amino acid, characterised in that the conversion is carried out by means of the microorganisms DSM 7329 and/or DSM 7330.

**4.** Process according to claim 3, characterised in that the conversion is carried out with dormant microorganisms.

**5.** Use of the microorganisms DSM 7329 and/or DSM 7330 for cultivating mutants or variants of these microorganisms.

## Revendications

**1.** Micro-organisme DSM 7329.

**2.** Micro-organisme DSM 7330.

**3.** Procédé pour la production de L-α-aminoacides par réaction enzymatique d'une D-hydantoïne monosubstituée en 5ème position, d'une L-hydantoïne monosubstituée en 5ème position et/ou d'une D,L-hydantoïne monosubstituée en 5ème position et/ou d'un D-N-carbamoyl-α-aminoacide, d'un L-N-carbamoyl-α-aminoacide et/ou d'un D,L-N-carbamoyl-α-aminoacide, caractérisé en ce que la réaction est réalisée à l'aide du ou des micro-organismes DSM 7329 et/ou DSM 7330.

**4.** Procédé selon la revendication 3, caractérisé en ce que la réaction est réalisée avec des micro-organismes au repos.

**5.** Utilisation du ou des micro-organismes DSM 7329 et/ou DSM 7330 pour la culture de mutants ou de variantes de ces micro-organismes.